(19) Europäisches Patentamt
European Patent Office
Office européen des brevets

(11) EP 2 011 785 B1

(12) **EUROPEAN PATENT SPECIFICATION**

(45) Date of publication and mention of the grant of the patent:
**18.12.2013 Bulletin 2013/51**

(21) Application number: **07737055.9**

(22) Date of filing: **12.04.2007**

(51) Int Cl.:
***C07C 319/14*** *(2006.01)* ***C08G 18/76*** *(2006.01)*
***C08G 18/38*** *(2006.01)*

(86) International application number:
**PCT/JP2007/000398**

(87) International publication number:
**WO 2007/129449 (15.11.2007 Gazette 2007/46)**

# (54) METHOD FOR PRODUCING POLYTHIOL COMPOUND FOR OPTICAL MATERIAL

VERFAHREN ZUR HERSTELLUNG EINER POLYTHIOLVERBINDUNG FÜR OPTISCHES MATERIAL

PROCÉDÉ DE PRODUCTION D'UN COMPOSÉ POLYTHIOL POUR MATÉRIAU OPTIQUE

(84) Designated Contracting States:
**DE FR GB IT**

(30) Priority: **20.04.2006 JP 2006116459**

(43) Date of publication of application:
**07.01.2009 Bulletin 2009/02**

(73) Proprietor: **Mitsui Chemicals, Inc.**
**Tokyo 105-7117 (JP)**

(72) Inventors:
- **KUMA, Shigetoshi**
  **Omuta-shi, Fukuoka 8368610 (JP)**
- **KAWAGUCHI, Masaru**
  **Tokyo 1057117 (JP)**
- **KOBAYASHI, Seiichi**
  **Omuta-shi, Fukuoka 8368610 (JP)**

(74) Representative: **Wills, Andrew Jonathan**
**Mewburn Ellis LLP**
**33 Gutter Lane**
**London**
**EC2V 8AS (GB)**

(56) References cited:
**JP-A- 02 270 859** **JP-A- 05 208 950**
**JP-A- 09 286 772**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 011 785 B1

## Description

### TECHNICAL FIELD

The present invention relates to a process for producing a polythiol compound for an optical material, and a polymerizable composition containing the polythiol compound and a polyiso (thio) cyanate compound, which can be used for a polyurethane resin providing good optical properties.

### BACKGROUND ART

Plastic lenses are light weight, less broken, and dyeable, as compared with inorganic lenses. Therefore, in recent years, the application of the plastic lenses to optical materials for a spectacle lens, or a camera lens, has increased rapidly.

The resins for the plastic lenses have been required to have new excellent performances such as a high refractive index, a high Abbe's number, low specific gravity, and high heat resistance. A variety of resin materials for lenses have been developed and used until now.

Among them, there have been actively proposed polythiourethane-based resins, and the present inventors have been also proposed various plastic lenses obtained by using these polythiourethane-based resins (see Patent Documents 1, 2, and 3).

Among the polythiourethane-based resins, as a representative example of such resins, a resin obtained by reacting a polythiol with a polyiso(thio)cyanate compound is one of the resins that have colorless and transparent, a high refractive index, and a low dispersion, and are suitable for plastic lenses having excellent impact resistance, dyeabiltiy, and processibility. Among these, the transparency of the resin is an essential property for lens.

[Patent Document 1] Japanese Patent Laid-open No.H2-270859 (EP 0378895)
[Patent Document 2] Japanese Patent Laid-open No.H9-110955 (EP 0528590)
[Patent Document 3] Japanese Patent Laid-open No.H9-110956

### DISCLOSURE OF THE INVENTION

The resins obtained as described in the above-described documents are required, if applied for an optical material, to be less colored, and transparent. Various methods may be applied in order to improve the color of the resin, but usually the color of the resin is likely to be affected by the color of the polythiol to be polymerized. When the polythiol is colored, the obtained resin may have poor color in some cases. Thus, it is often preferable that the coloration of the polythiol to be polymerized be suppressed so as to obtain a less colored and transparent resin. From an industrial viewpoint, it has been required that those with good color are constantly produced and thereby not producing products with poor color. For this reason, there has been demanded a method for constantly preparing a polythiol compound and a polyurethane resin, with high transparency and less coloration.

The present inventors have conducted extensive studies to solve the above-described problems, and as a result, they have found that deterioration of the color of the polyurethane resin is due to the impurities in 2-mercaptoethanol that is a starting material for preparing a polythiol. Further, the present inventors have conducted further extensive studies, and as a result, they have found that if the content of the bis(2-hydroxyethyl) disulfide in 2-mercaptoethanol that is a starting material for preparing a polythiol is more than a specific value, the color of the polythiol is deteriorated, and the color of the resin obtained by using the polythiol having poor color is also deteriorated. Accordingly, they have found that the above-described problems are solved by preparing a polythiol using 2-mercaptoethanol, in which the content of the bis(2-hydroxyethyl) disulfide is no more than a given value, as a starting material, and thus a polythiol compound and a polyurethane resin, each having a good color can be obtained, thereby completing the present invention.

That is, the present invention includes the follows.

(1) a process for producing a polythiol compound of Formula (3) for an optical material, comprising:

reacting 2-mercaptoethanol with an epihalohydrin compound represented by following Formula (1) to obtain a polyalcohol represented by following Formula (2) with a thiourea to obtain a thiouronium salt, and hydrolyzing the thiouronium salt to produce a polythiol compound represented by following Formula (3), wherein the content of bis(2-hydroxyethyl) disulfide in 2-mercaptoethanol is 0.5 wt% or less and an aqueous ammonia is used in said hydrolyzing the thiouronium.

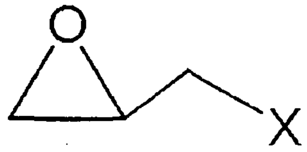

(1)

In Formula (1), X represents a halogen atom.

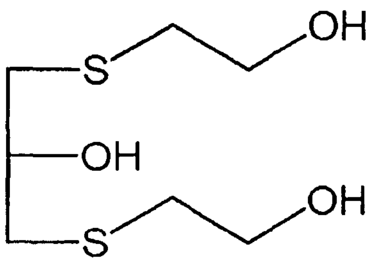

(2)

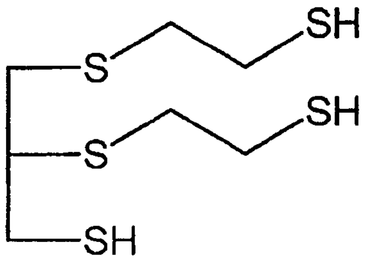

(3)

(2) the process for producing a polythiol compound for an optical material as described in (1) wherein X in Formula (1) is a chlorine atom.

The invention provides a method for preparing a polythiol compound for an optical material having a good color, and thus contributes the development in the relevant field.

BEST MODE FOR CARRYING OUT THE INVENTION

Hereinbelow, the present invention will be described in detail. 2-mercaptoethanol as a starting material for the polythiol compound of Formula (3) has a content of bis(2-hydroxyethyl) disulfide of no more than a specific amount. When the amount is no more than a given amount, it is not particularly limited, but the content of bis(2-hydroxyethyl) disulfide in 2-mercaptoethanol is preferably in a range of 0.01 wt% to 0.5 wt% both inclusive, more preferably in a range of 0.01 wt% to 0.45 wt% both inclusive, and even more preferably in a range of 0.01 wt% to 0.35 wt% both inclusive.

When the content of bis(2-hydroxyethyl) disulfide is 0.5 wt% or less, a polythiol compound represented by Formula (3) and a polyurethane resin, each having a good color, can be obtained. The content of the bis (2-hydroxyethyl) disulfide can be measured by high performance liquid chromatography.

2-Mercaptoethanol is oxidized with oxygen to produce bis (2-hydroxyethyl) disulfide. Alternatively, if a metal component such as iron exists or the temperature condition is elevated, the production rate increases. Thus, it is preferable that 2-mercaptoethanol is stored until the use in the reaction, at 30°C or lower, in a vessel containing no metal component, under an inert gas such as nitrogen and argon while blocking air. Alternatively, 2-mercaptoethanol is isolated and purified by distillation, column chromatography, to reduce the content of bis(2-hydroxyethyl) disulfide.

The process for producing the polythiol compound for an optical material represented by Formula (3) of the present invention is as follows.

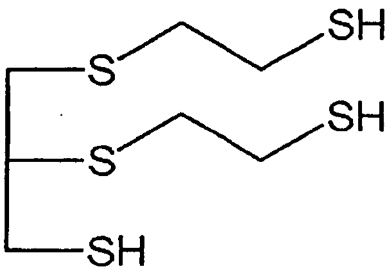

(3)

An epihalohydrin compound represented by Formula (1) is reacted with 2-mercaptoethanol in the presence of an alkali under cooling or heating, to obtain a polyalcohol represented by Formula (2).

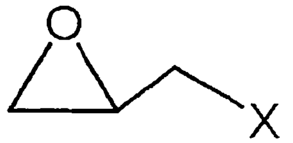

(1)

In the Formula (1), X represents a halogen atom.

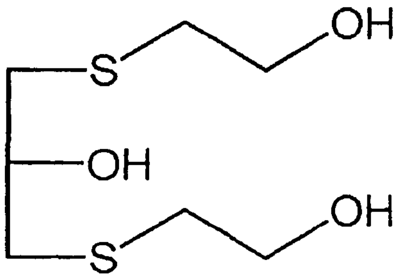

(2)

Then, the polyalcohol represented by Formula (2) is reacted with a thiourea in a mineral acid, and hydrolyzed in the presence of aqueous ammonia to form a thiol group. When the mineral acid and the thiourea are added, rearrangement can occur in the 1 and 2 positions in a propane skeleton to obtain a polythiol represented by Formula (3).

In the compound of Formula (1), X is a halogen atom, preferably a chlorine atom. As the compound of Formula (1), preferred is epichlorohydrin.

Specifically, the method for reacting the 2-mercaptoethanol with the compound of Formula (1) is, for example, as follows. First, 2-mercaptoethanol and a base are added to water or a lower alcohol solvent such as methanol and ethanol, and then epichlorohydrin is added dropwise thereto. At this time, the reaction is preferably carried out at a reaction temperature of 10 to 100°C. The amount of 2-mercaptoethanol to be used is 2 to 5 equivalents, preferably 2 to 3 equivalents, based on epichlorohydrin. Examples of the base include a metal hydroxide such as sodium hydroxide and potassium hydroxide, a metal carbonate such as sodium carbonate and potassium carbonate, and a tertiary amine such as triethyl amine and tributyl amine, but from the viewpoints of reactivity and economical production, most preferred is a sodium hydroxide. The amount of the base to be used is 1 to 5 equivalents both inclusive, based on epichlorohydrin, but is preferably no more than moles of 2-mercaptoethanol to be used.

Furthermore, the reaction of 2-mercaptoethanol with the compound represented by Formula (1) may be carried out by a method which includes dropwisely adding a base of 1 to 1. 5 equivalent amount to 2-mercaptoethanol based on 2-mercaptoethanol, and then adding epichlorohydrin dropwise thereinto after forming a salt, in order to control the coloration of thiol. This method is performed through a diol represented by Formula (4).

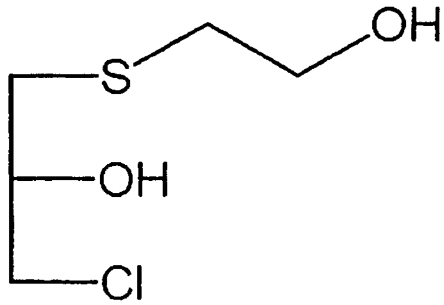

(4)

When the method is performed through the diol represented by Formula (4), epichlorohydrin is added dropwise to an aqueous solution of 2-mercaptoethanol and above-mentioned base or a solution of 2-mercaptoethanol and above-mentioned base and a lower alcohol such as methanol and ethanol. In the solution to which epichlorohydrin is added dropwise, the amount of 2-mercaptoethanol to be used is in a range of 1 to 3 equivalents both inclusive, preferably in a range of 1 to 2 equivalents both inclusive, based on epichlorohydrin. Further, a catalytic amount of the base is used, and the amount of the base to be used is preferably in a range of 0.001 to 0.1 equivalent both inclusive, based on epichlorohydrin. By adding epichlorohydrin dropwise to the solution, a diol represented by Formula (4) can be obtained. Subsequently, 2-mercaptoethanol may be further added if the amount is not enough to be 2 to 3 equivalents both inclusive to that of the epichlorohydrin, and also the base may be further added if the amount is not enough to be 1 to 2 equivalents both inclusive to that of epichlorohydrin, thereby obtaining a polyalcohol represented by Formula (2). When a strong base such as sodium hydroxide is used in the synthesis of the diol represented by Formula (4), the reaction temperature is suitably 0 to 50°C both inclusive. When the reaction temperature is too high, the base added in a catalytic amount is consumed in the reaction for production of a polyalcohol from the diol, thereby lowering the yield of the diol product. When a tertiary amine is used in the synthesis of the diol, such the problem does not occur even at a reaction temperature of 50 to 120°C both inclusive.

Next, the method for producing a polythiol compound after obtaining the polyalcohol will be specifically described. 3 equivalents or more, preferably 3 to 6 equivalents of a thiourea is added to the polyalcohol represented by Formula (2). They are reacted with each other in an aqueous solution of mineral acid which is a 3 equivalents or more, preferably 3 to 12 equivalents, based on the polyalcohol, at a temperature in a range of room temperature to a reflux temperature. The polyalcohol is reacted with the thiourea to prepare a thiouronium salt.

As the mineral acid, hydrochloric acid, hydrobromic acid, hydroiodic acid, sulfuric acid, or phosphoric acid, can be used, but preferred is hydrochloric acid, from a viewpoint that it is capable to give a sufficient reaction rate, and allows the coloration of a product to be controlled.

Subsequently, the hydrolysis reaction is carried out by adding 1 equivalent or more, preferably 1 to 3 equivalents, and more preferably 1.1 to 2 equivalents both inclusive of ammonia, with respect to the amount of said mineral acid used to above-mentioned reaction solution so as to make the solution alkaline, at a temperature in a range of room temperature to a reflux temperature. The temperature upon addition of the base is preferably 0 to 50°C both inclusive. When the temperature is within said range, the coloration of the product hardly occurs.

The polythiol compound for an optical material represented by Formula (3), thus produced, can be extracted from an organic solvent such as toluene, and then purified by acid washing, water washing, concentration, or filtration, and if necessary, purified with distillation.

Furthermore, the process of the present invention can be practiced even in the air, but it is preferable that it is entirely practiced under a nitrogen atmosphere from the viewpoint of the color.

The optical material for a polyurethane lens can be obtained by reacting the polythiol compound for an optical material represented by Formula (3) and a polyiso(thio)cyanate compound.

The polyiso(thio)cyanate compound that can be used is not particularly limited as long as it is compound having at least two iso (thio) cyanate groups in one molecule, and but specific examples thereof include aliphatic polyisocyanate compounds such as hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylene triisocyanate, 1,8-diisocyanato-4-isocyanatomethyloctane, bis(isocyanatoethyl) carbonate, bis(isocyanatoethyl) ether, lysine diisocyanatomethyl ester, and lysine triisocyanate;

polyisocyanate compounds having an aromatic group, such as 1,2-diisocyanatobenzene, 1,3-diisocyanatobenzene, 1,4-diisocyanatobenzene, 2,4-diisocyanatotoluene, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluidine diisocyanate, 4,4'-methylene bis(phenylisocyanate), 4,4'-methylene bis(2-methylphenylisocyanate), bibenzyl-4,4'-diisocyanate, bis(isocyanatophenyl)ethylene, bis(isocyanatomethyl)benzene, bis(isocyanatoethyl)benzene, bis(isocyanatopropyl)benzene, a,a,a',a'-tetramethylxylylene diisocyanate, bis(isocyanatobutyl)benzene, bis(isocyanatomethyl)naphthalene, bis(isocyanatomethylphenyl)ether, bis(isocyanatoethyl)phthalate, and 2,6-di(isocyanatomethyl) furane;
sulfur-containing aliphatic polyisocyanate compounds such as bis(isocyanatomethyl) sulfide, bis(isocyanatoethyl) sulfide, bis(isocyanatopropyl) sulfide, bis(isocyanatohexyl) sulfide, bis(isocyanatomethyl) sulfone, bis(isocyanatomethyl) disulfide, bis(isocyanatoethyl) disulfide, bis(isocyanatopropyl) disulfide, bis(isocyanatomethylthio)methane, bis (isocyanatoethylthio)methane, bis(isocyanatomethylthio)ethane, bis(isocyanatoethylthio)ethane, 1,5-diisocyanato-2-isocyanatomethyl-3-thiapentane, 1,2,3-tris(isocyanatomethylthio)propane, 1,2,3-tris(isocyanatoethylthio)propane, 3,5-dithia-1,2,6,7-heptane tetraisocyanate, 2,6-diisocyanatomethyl-3,5-dithia-1,7-heptane diisocyanate, 2,5-diisocyanatomethylthiophene, and 4-isocyanatoethylthio-2,6-dithia-1,8-octanediisocyanate;
aromatic sulfide polyisocyanate compounds such as 2-isocyanatophenyl-4-isocyanatophenyl sulfide, bis(4-isocyanatophenyl) sulfide and bis(4-isocyanatomethylphenyl) sulfide;
aromatic disulfide polyisocyanate compounds such as bis(4-isocyanatophenyl) disulfide, bis(2-methyl-5-isocyanatophenyl) disulfide, bis(3-methyl-5-isocyanatophenyl) disulfide, bis(3-methyl-6-isocyanatophenyl) disulfide, bis(4-methyl-5-isocyanatophenyl) disulfide, and bis(4-methoxy-3-isocyanatophenyl) disulfide;
sulfur-containing alicyclic polyisocyanate compounds such as 2,5-diisocyanatotetrahydrothiophene, 2,5-diisocyanatomethyltetrahydrothiophene, 3,4-diisocyanatomethyltetrahydrothiophene, 2,5-diisocyanato-1,4-dithiane, 2,5-diisocyanatomethyl-1,4-dithiane, 4,5-diisocyanato-1,3-dithiolane, 4,5-bis(isocyanatomethyl)-1,3-dithiolane, and 4,5-diisocyanatomethyl-2-methyl-1,3-dithiolane;
aliphatic polyisothiocyanate compounds such as 1,2-diisothiocyanatoethane and 1,6-diisothiocyanatohexane;
alicyclic polyisothiocyanate compounds such as cyclohexanediisothiocyanate;
aromatic polyisothiocyanate compounds such as 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-methylene bis(phenylisothiocyanate), 4,4'-methylene bis(2-methylphenylisothiocyanate), 4,4'-methylene bis(3-methylphenylisothiocyanate), 4,4'-diisothiocyanatobenzophenone, 4,4'-diisothiocyanato-3,3'-dimethylbenzophenone, and bis(4-isothiocyanatophenyl) ether;
carbonylpolyisothiocyanate compounds such as 1,3-benzenedicarbonyl diisothiocyanate, 1,4-benzenedicarbonyl diisothiocyanate, and (2,2-pyridine)-4,4-dicarbonyl diisothiocyanate;
sulfur-containing aliphatic polyisothiocyanate compounds such as thiobis(3-isothiocyanatopropane), thiobis(2-isothiocyanatoethane), and dithiobis(2-isothiocyanatoethane);
sulfur-containing aromatic polyisothiocyanate compounds such as 1-isothiocyanato-4-[(2-isothiocyanato)-sulfonyl]benzene, thiobis(4-isothiocyanatobenzene), sulfonyl(4-isothiocyanatobenzene), and dithiobis(4-isothiocyanatobenzene);
sulfur-containing alicyclic polyisothiocyanate compounds such as 2,5-diisothiocyanatothiophene and 2,5-diisothiocyanato-1,4-dithiane; and
compounds having isocyanate and isothiocyanate groups such as 1-isocyanato-6-isothiocyanatohexane, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanatophenyl-4-isothiocyanatophenyl sulfide, and 2-isocyanatoethyl-2-isothiocyanatoethyl disulfide.

Furthermore, there may be used a halogen substitute such as a chlorine substitute or a bromine substitute, an alkyl substitute, an alkoxy substitute, a nitro substitute, polyhydric alcohol prepolymer-modified products, carbodiimide-modified products, urea-modified products, burette-modified products, or dimerization or trimerization reaction products, of those compounds. These compounds may be used singly, or in combination of two or more kinds thereof.

A proportion of the polythiol compound to the polyiso (thio) cyanate to be used is not particularly limited, but is usually for the molar ratio, SH group/NCO group, to be in the range of from 0.5 to 3.0 both inclusive, preferably from 0.6 to 2.0 both inclusive, more preferably from 0.8 to 1.3 both inclusive. When the ratio is within above range, it is possible to obtain a good balance among the various performances such as a refractive index and heat resistance, which are required for optical materials and transparent materials for a plastic lens.

For the purpose of improving various physical properties, operability and polymerization reactivity of the polyurethane resin described herein, other materials, in addition to the polythiol compound and the iso (thio) cyanate compound for forming an urethane resin, can also be added. For example, one, or two or more kinds of an active hydrogen compound such as an amine, and an epoxy compound, an olefin compound, a carbonate compound, an ester compound, a metal, a metal oxide, an organic metal compound, and an inorganic substance, can be further incorporated.

Further, a variety of substances such as a chain extender, a crosslinking agent, a photostabilizer, a UV absorber, an antioxidant, an oil soluble dye, a filler, a releasing agent, and a blueing agent, may be added, depending on the purposes, as in a known molding method. In order to adjust to a desired reaction rate, a thiocarbamic acid S-alkyl ester or a known reaction catalyst used for producing polyurethane may be added as appropriate. The lens formed of the polyurethane resin can be usually obtained by casting polymerization.

Specifically, the polythiol compound obtained by the preparation method of the present invention, and the polyiso (thio) cyanate compound can be mixed to obtain a mixed solution. This mixture is, if necessary, degassed by

means of an appropriate method, injected to a mold, and then usually heated gradually from a lower temperature to a higher temperature, thereby performing the polymerization.

As such, the polyurethane resin that can be obtained by curing the polymerizable composition described herein has a high refractive index, a low dispersion, excellent heat resistance and durability, light weight, and excellent impact resistance, as well as good color, thereby it being suitable as an optical material and a transparent material for a spectacle lens, or a camera lens.

Furthermore, the lens which is obtained by using the polyurethane resin described herein may be, if necessary, subjected to physical or chemical treatment such as surface abrasion treatment, antistatic treatment, hard coat treatment, non-reflective coat treatment, dyeing treatment and polarizing treatment, for prevention of reflection, enhancement of hardness, improvement of abrasion resistance, improvement of chemical resistance, supply of anticlouding and supply of fashionability.

[Examples]

Hereinafter, the present invention will be further specifically described with reference to Examples. Analysis of the 2-mercaptoethanol used was carried out in the following methods. The Y. I. values of the polythiol and the resin, thus obtained, were determined by the following test methods. Further, APHA was evaluated by the following test methods.

- Content of bis (2-hydroxyethyl) disulfide: 2-mercaptoethanol was dissolved in water, and the content thereof was measured by high performance liquid chromatography.
- Color of polythiol (Y. I. Value): The Y. I. value as used is a yellow index in the color evaluation, and is a value that can be measured by a colorimeter. A smaller Y. I. value indicates a better color. A colorimeter manufactured by Minolta Co., Ltd. (CR-200, CT-210) was used to measure the Y. I. value. First, distilled water was added into a 20 mm-light path cell CT-A20 for white calibration with Y=100.00, x=0.3101, and y=0.3162. Then, a sample was put into the same cell thereby determining the yellow index (Y. I.). Based on the resulting x and y values, the following equation was used to determine the Y. I.

$$Y.\ I. = (234x+106y+106)/y \qquad (1)$$

The color of the polythiol is represented by the Y. I. Value.

- Color of plastic lens obtained from polyurethane resin (Y. I. Value): A colorimeter, CT-210, manufactured by Minolta Co., Ltd. was used to measure the Y. I. of a plastic lens obtained from the polyurethane resin. A round flat plate with a thickness of 9 mm and $\phi$ of 75 mm was produced by cast polymerization, and then measured on the color coordination, x and y. Based on the resulting x and y values, the above equation (1) was used to determine the yellow index (Y. I).

APHA: The APHA in the present invention is indicative of colors. APHA was obtained by comparing the color of a sample to diluted standard solution having an equivalent concentration using a standard solution prepared by melting a reagent of platinum and cobalt. Its degree was taken as a measurement value. The smaller the value was, the better the color was.

[Example 1]

(Synthesis of polythiol having

1,2-bis[(2-mercaptoethyl)thio]-3-mercaptopropane as main component)

Into a 2-L four-neck reaction flask equipped with a stirrer, a reflux condenser with cooling water, a nitrogen gas purging pipe, and a thermometer, 169 parts by weight (2.16 mol) of 2-mercaptoethanol which has a purity of 99.80%, and contains 0.13 wt% of bis(2-hydroxyethyl) disulfide, which that had been obtained by distillation at an ambient pressure and stored in a glass-made vessel under a nitrogen stream, and 76.0 parts by weight of water were charged. At 30°C, 91.9 parts by weight (1.08 mol) of a 47 wt% aqueous sodium hydroxide solution was charged dropwise thereinto over 30 minutes, and then 99.9 parts by weight (1.08 mol) of epichlorohydrin was charged thereinto at the same temperature over 3 hours, and the mixture was aged for 1 hour. Then, 450.0 parts by weight (4.32 mol) of a 35 wt% HCl solution, and 246.9 parts by weight (3.24 mol) of thiourea having a purity of 99.90% were charged thereinto, and the mixture was

aged under reflux at 110°C over 3 hours, thereby making a thiouronium salt. Then, the mixture was cooled to 60°C, then 450.0 parts by weight of toluene, and 331.1 parts by weight (4.86 mol) of a 25 wt% aqueous ammonia solution were charged into the mixture, to carry out hydrolysis, thereby obtaining a solution of a polythiol in toluene, having 1,2-bis[(2-mercaptoethyl)thio]-3-mercaptopropane as a main component. The toluene solution was washed with an acid and then water, heated under reduced pressure to remove toluene and a trace amount of moisture. Then, the residue was filtered to obtain 268.7 parts by weight of a polythiol having 1,2-bis[(2-mercaptoethyl)thio]-3-mercaptopropane as a main component. The obtained polythiol had an APHA of 10, and a Y. I value of 0.92.

(Preparation of plastic lens)

52 parts by weight of m-xylylene diisocyanate, 0.015 part by weight of dibutyltin dichloride as a curing catalyst, 0.10 part by weight of "Zelec UN" (trade name, acidic alkyl phosphate) manufactured by Stepan Co. Ltd., and 0.05 part by weight of "Viosorb 583" (trade name, a UV absorber) manufactured by Kyodo Yakuhin Co. Ltd. were mixed and dissolved at 20°C. 48 parts by weight of the obtained polythiol having 1,2-bis[(2-mercaptoethyl)thio]-3-mercaptopropane as a main component was charged thereinto to give a homogeneous mixed solution. This homogeneous solution was degassed at 600 Pa for 1 hour. Then, the residue was filtered through a 1 μm PTFE filter, and then charged into a mold comprising a glass mold and tape. The mold was placed in an oven, and slowly heated from 10°C to 120°C to perform the polymerization over 20 hours. After completing the polymerization, the mold was taken out from the oven, and mold-released to obtain a resin. The obtained resin was further subjected to annealing at 120°C for 3 hours. The obtained resin had a Y. I value of 4.7. The results are shown in Table 1.

[Example 2]

A polythiol having 1,2-bis[(2-mercaptoethyl)thio]-3-mercaptopropane as a main component was synthesized in the same manner as in Example 1, except that 2-mercaptoethanol with a purity of 99.40%, containing 0.32 wt% of bis(2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 10, and a Y. I value of 0.94. A plastic lens was prepared using the polythiol, and then evaluated, in the same manner as in Example 1. The evaluation results of the obtained plastic lens are shown in Table 1.

[Example 3]

A polythiol having 1,2-bis[(2-mercaptoethyl)thio]-3-mercaptopropane as a main component was synthesized in the same manner as in Example 1, except that 2-mercaptoethanol with a purity of 99.17%, containing 0.45 wt% of bis(2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 10, and a Y. I value of 1.23. A plastic lens was prepared using the polythiol, and then evaluated, in the same manner as in Example 1. The evaluation results of the obtained plastic lens are shown in Table 1.

[Comparative Example 1]

A polythiol having 1,2-bis[(2-mercaptoethyl)thio]-3-mercaptopropane as a main component was synthesized in the same manner as in Example 1, except that 2-mercaptoethanol with a purity of 99.10%, containing 0.75 wt% of bis(2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 15, and a Y. I value of 1.88. A plastic lens was prepared using the polythiol, and then evaluated, in the same manner as in Example 1. The evaluation results of the obtained plastic lens are shown in Table 1.

[Comparative Example 2]

A polythiol having 1,2-bis[(2-mercaptoethyl)thio]-3-mercaptopropane as a main component was synthesized in the same manner as in Example 1, except that 2-mercaptoethanol with a purity of 98.80%, containing 0. 90 wt% of bis(2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 20, and a Y. I value of 2.34. A plastic lens was prepared using the polythiol, and then evaluated, in the same manner as in Example 1. The evaluation results of the obtained plastic lens are shown in Table 1.

[Comparative Example 3]

A polythiol having 1,2-bis[(2-mercaptoethyl)thio]-3-mercaptopropane as a main component was synthesized in the same manner as in Example 1, except that 2-mercaptoethanol with a purity of 99.0%, containing 0.65 wt% of bis (2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 10, and a Y. I value of 1.60. A plastic lens was prepared using the polythiol, and then evaluated, in the same manner as in Example 1. The evaluation results of the obtained plastic lens are shown in Table 1.

[Comparative Example 4]

A polythiol having 1,2-bis[(2-mercaptoethyl)thio]-3-mercaptopropane as a main component was synthesized in the same manner as in Example 1, except that 2-mercaptoethanol with a purity of 99.20%, containing 0.52 wt% of bis(2-hydroxyethyl) disulfide, was used instead of 2-mercaptoethanol used in Example 1. The obtained polythiol having 1,2-bis[(2-mercaptoethyl)thio]-3-mercaptopropane as a main component had an APHA of 10, and a Y. I value of 1.45. A plastic lens was prepared using the polythiol, and then evaluated, in the same manner as in Example 1. The evaluation results of the obtained plastic lens are shown in Table 1.

[Comparative Example 5]

A polythiol having 1,2-bis[(2-mercaptoethyl)thio]-3-mercaptopropane as a main component was synthesized in the same manner as in Example 1, except that 2-mercaptoethanol with a purity of 99.20%, containing 0.52 wt% of bis(2-hydroxyethyl) disulfide, was used instead of 2-mercaptoethanol used in Example 1, and 354.8 parts by weight (4.32 mol) of a 48.7 wt% sodium hydroxide solution was used instead of 331. 1 parts by weight (4.86 mol) of the 25 wt% aqueous ammonia solution used in Example 1. The obtained polythiol having 1,2-bis[(2-mercaptoethyl)thio]-3-mercaptopropane as a main component had an APHA of 10, and a Y. I value of 1.60. A plastic lens was prepared using the polythiol, and then evaluated, in the same manner as in Example 1. The evaluation results of the obtained plastic lens are shown in Table 1.

(Table 1)

| | -S-S- (wt%)* | Color of polythiol | | Color of resin |
|---|---|---|---|---|
| | | APHA | Y. I. | Y. I. |
| Example 1 | 0.13 | 10 | 0.92 | 4.7 |
| Example 2 | 0.32 | 10 | 0.94 | 4.8 |
| Example 3 | 0.45 | 10 | 1.23 | 5.1 |
| Comparative Example 1 | 0.75 | 15 | 1.88 | 6.0 |
| Comparative Example 2 | 0.90 | 20 | 2.34 | 6.5 |
| Comparative Example 3 | 0.65 | 10 | 1.60 | 5.6 |
| Comparative Example 4 | 0.52 | 10 | 1.45 | 5.3 |
| Comparative Example 5 | 0.52 | 10 | 1.60 | 6.1 |
| * Content of bis(2-hydroxyethyl) disulfide in 2-mercaptoethanol | | | | |

The resins obtained in Examples and Comparative Examples were individually transparent without coloration, but upon comparison between the resins of Examples and the resins of Comparative Examples, the resins of Comparative Examples were more yellowish than those in Examples.
In the results of Examples and Comparative Examples, it is confirmed that in proportion to the content of bis(2-hydroxyethyl) disulfide in 2-mercaptoethanol that is a starting material, the color of the resulting polythiol compound is deteriorated, and accordingly, the color of the resin is also deteriorated. Further, it was proved that the color of polythiol and the resin tends to deteriorate significantly if the content of bis(2-hydroxyethyl) disulfide is greater than 0.5 wt%. That is, it was confirmed that by using 2-mercaptoethanol having a content of bis (2-hydroxyethyl) disulfide of 0.5 wt% or less, a polythiol compound and a polyurethane resin,

each having a good color with its coloration suppressed, can be provided. Further, it could be found that a polythiol compound and a polyurethane resin, each having a better color, can be provided by using aqueous ammonia in the hydrolysis of a thiouronium salt.

[Industrial Availability]

A polyurethane resin obtained by the reaction of a polythiol compound with a polyiso(thio)cyanate compound is one of the resins which has colorless and transparent, a high refractive index, and a low dispersion, and is most suited as a transparent material or an optical material for plastic lenses having excellent impact resistance, dyeabiltiy, and processibility. Among these, the resin is required to have properties such as transparency and good color. The present reliably provides a method for preparing a polythiol compound for an optical material and a transparent material, and a polyurethane lens, each having properties required as a plastic lens, and thus contributes to development in the relevant field.

## Claims

1. A process for producing a polythiol compound of Formula (3) for an optical material, comprising:

   reacting 2-mercaptoethanol with an epihalohydrin compound represented by following Formula (1) to obtain a polyalcohol represented by following Formula (2), reacting the polyalcohol represented by Formula (2) with a thiourea to obtain a thiouronium salt, and
   hydrolyzing the thiouronium salt to produce a polythiol compound represented by following Formula (3),
   wherein the content of bis(2-hydroxyethyl) disulfide in 2-mercaptoethanol is 0.5 wt % or less and
   wherein an aqueous ammonia is used in said hydrolyzing the thiouronium salt:

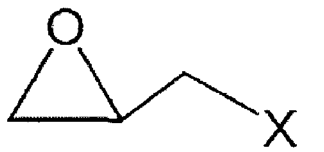
(1)

   wherein , X represents a halogen atom

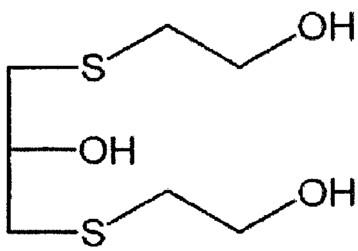
(2)

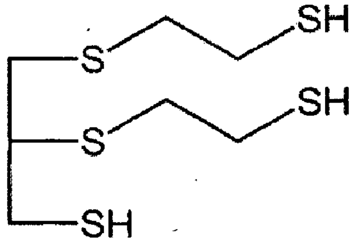
(3)

2. The process for producing a polythiol compound for an optical material as claimed in claim 1, wherein X in said Formula (1) is a chlorine atom.

## Patentansprüche

1. Verfahren zur Herstellung einer Polythiolverbindung der Formel (3) für ein optisches Material, Folgendes umfassend:

 das Umsetzen von 2-Mercaptoethanol mit einer durch die nachstehende Formel (1) dargestellten Epihalohydrinverbindung, um einen durch die nachstehende Formel (2) dargestellten Polyalkohol zu erhalten, das Umsetzen des durch die Formel (2) dargestellten Polyalkohols mit einem Thioharnstoff, um eine Thiouroniumsalz zu erhalten, und
 das Hydrolysieren des Thiouroniumsalzes, um eine durch die nachstehende Formel (3) dargestellte Polythiolverbindung zu erhalten,
 worin der Gehalt von Bis-(2-hydroxyethyl)disulfid in 2-Mercaptoethanol 0,5 Gew.-% oder weniger beträgt und worin beim Hydrolysieren des Thiouroniumsalzes wässriges Ammoniak verwendet wird:

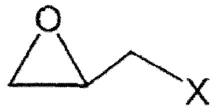
(1)

 worin X für ein Halogenatom steht,

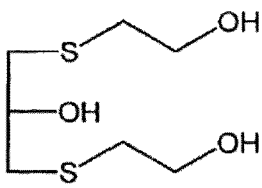
(2)

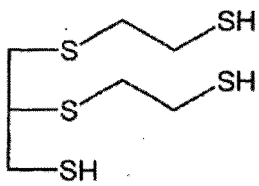
(3)

2. Verfahren zur Herstellung einer Polythiolverbindung für ein optisches Material nach Anspruch 1, worin in der Formel (1) X ein Chloratom ist.

## Revendications

1. Procédé de production d'un composé polythiol de la Formule (3) pour un matériau optique, comprenant :

 faire réagir 2-mercaptoéthanol avec un composé épihalohydrine représenté par la Formule suivante (1) pour obtenir un polyalcool représenté par la Formule suivante (2), faire réagir le polyalcool représenté par la Formule (2) avec une thiourée pour obtenir un sel de thio-uronium, et
 hydrolyser le sel de thio-uronium pour produire un composé polythiol représenté par la Formule suivante (3),
 où la teneur en bis(2-hydroxyéthyl)disulfure dans 2-mercapto-éthanol est de 0,5 % en poids ou moins et
 où un ammoniac aqueux est utilisé dans ladite hydrolyse du sel de thio-uronium :

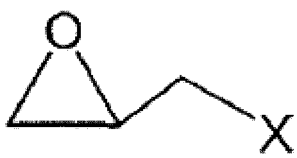
(1)

où X représente un atome d'halogène

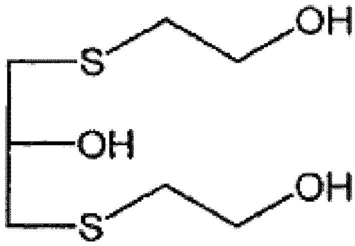

(2)

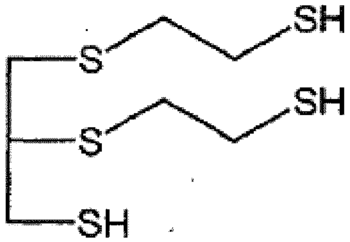

(3)

2. Procédé de production d'un composé de polythiol pour un matériau optique tel que revendiqué dans la revendication 1, où X dans ladite Formule (1) est un atome de chlor.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H2270859 B
- EP 0378895 A
- JP H9110955 B
- EP 0528590 A
- JP H9110956 B